# EUROPEAN PATENT APPLICATION

(11) **EP 4 717 284 A1**
(43) Date of publication of application: **01.04.2026**
(21) Application number: 24202463.6
(22) Date of filing: 25.09.2024
(51) Int. Cl.: A61M 1/00

(54) **NEGATIVE PRESSURE WOUND THERAPY**

(71) Applicant: Mölnlycke Health Care AB, 402 52 Göteborg (SE)
(72) Inventor: VANNAS, Alexander, 421 51 Göteborg (SE); BENGTSSON, Erik, 431 41 Mölndal (SE); UVEBORN, Johan, 436 40 Askim (SE)
(74) Representative: Zacco Sweden AB

(57) **Abstract**

An apparatus and related aspects for providing reduced pressure to a tissue site are disclosed. The apparatus comprises a source of negative pressure configured to provide negative pressure via a first fluid flow path from an inlet of the source of negative pressure to a wound site, power supply circuitry comprising a power source, the power supply circuitry being configured to provide a supply voltage to the source of negative pressure. The apparatus further comprises control circuitry operatively connected to the source of negative pressure and the power supply circuitry. The control circuitry is configured to, in response to receiving an indication that a noise level of ambient noise is above a noise level threshold, set the supply voltage to the source of negative pressure to a first voltage value above a nominal voltage value.

## Description

### TECHNICAL FIELD

The herein disclosed embodiments generally relate to negative pressure wound therapy (NPWT). In particular, the herein disclosed embodiments relate to apparatuses for providing reduced pressure to a wound site, wound treatment systems, methods for controlling an apparatus for providing reduced pressure to a wound site, and thereto related computer program products and computer-readable storage media.

### BACKGROUND

Negative pressure wound therapy (NPWT) is a technique that promotes healing of e.g. surgical, acute and chronic wounds by the application of a sub-atmospheric pressure ("negative pressure") to the wound site, using a negative pressure pump. Wound healing is achieved by applying a negative pressure, such as "vacuum" through a dressing or a cover applied onto the wound. Excess wound exudate is thereby drawn out, which increases the blood flow to the area, and promotes the formation of granulation tissue. The NPWT technique also permits less outside disturbance of the wound and transports excess fluids away from the wound site.

### SUMMARY

The herein disclosed technology seeks to mitigate, alleviate or eliminate one or more of the above-identified deficiencies and disadvantages in the prior art to address various problems relating to blockage detection in an NPWT system.

Various aspects and embodiments of the disclosed technology are defined below and in the accompanying independent and dependent claims.

An aspect of the disclosed technology comprises an apparatus for providing negative pressure to a wound site. The apparatus comprises a source of negative pressure configured to provide negative pressure via a first fluid flow path from an inlet of the source of negative pressure to a wound site, power supply circuitry comprising a power source, the power supply circuitry being configured to provide a supply voltage to the source of negative pressure. The apparatus further comprises control circuitry operatively connected to the source of negative pressure and the power supply circuitry. The control circuitry is configured to, in response to receiving an indication that a noise level of ambient noise is above a noise level threshold, set the supply voltage to the source of negative pressure to a first voltage value above a nominal voltage value. Here, the nominal voltage value may be a set voltage value to be provided to the source of negative pressure by the power supply circuitry when the noise level is below the threshold.

Another aspect of the disclosed technology comprises a method for operating an apparatus for providing negative pressure to a wound site. The apparatus comprises a source of negative pressure configured to provide negative pressure via a first fluid flow path from an inlet of the source of negative pressure to a wound site, power supply circuitry comprising a power source, the power supply circuitry being configured to provide a supply voltage to the source of negative pressure. The method comprises, in response to receiving an indication that a noise level of ambient noise is above a noise level threshold, setting the supply voltage to the source of negative pressure to a first voltage value above a nominal voltage value. With this aspect of the disclosed technology, similar advantages and preferred features are present as in the other aspects.

Another aspect of the disclosed technology comprises a computer program product comprising instructions which, when the program is executed by a computing device of an apparatus for providing reduced pressure to a wound dressing, causes the apparatus to carry out the method according to any one of the embodiments disclosed herein. With this aspect of the disclosed technology, similar advantages and preferred features are present as in the other aspects.

Another aspect of the disclosed technology comprises a (non-transitory) computer-readable storage medium comprising instructions which, when executed by a computing device of an apparatus for providing reduced pressure to a wound dressing, causes the apparatus to carry out the method according to any one of the embodiments disclosed herein. With this aspect of the disclosed technology, similar advantages and preferred features are present as in the other aspects.

The term "non-transitory," as used herein, is intended to describe a computer-readable storage medium (or "memory") excluding propagating electromagnetic signals, but are not intended to otherwise limit the type of physical computer-readable storage device that is encompassed by the phrase computer-readable medium or memory. For instance, the terms "non-transitory computer readable medium" or "tangible memory" are intended to encompass types of storage devices that do not necessarily store information permanently, including for example, random access memory (RAM). Program instructions and data stored on a tangible computer-accessible storage medium in non-transitory form may further be transmitted by transmission media or signals such as electrical, electromagnetic, or digital signals, which may be conveyed via a communication medium such as a network and/or a wireless link. Thus, the term "non-transitory", as used herein, is a limitation of the medium itself (i.e., tangible, not a signal) as opposed to a limitation on data storage persistency (e.g., RAM vs. ROM).

Another aspect of the disclosed technology comprises a wound treatment system comprising apparatus according to any one of the embodiments disclosed herein, a wound cover for creating a sealed space defined in part by the wound site, and a tubing assembly defining the fluid flow path. With this aspect of the disclosed technology, similar advantages and preferred features are present as in the other aspects.

The disclosed aspects and preferred embodiments may be suitably combined with each other in any manner apparent to anyone of ordinary skill in the art, such that one or more features or embodiments disclosed in relation to one aspect may also be considered to be disclosed in relation to another aspect or embodiment of another aspect.

An advantage of some embodiments the apparatus automatically adapts to the noise level in the surrounding environment of the patient/user, thereby improving the user experience.

An advantage of some embodiments is that the apparatus is allowed to operate more efficiently, by reducing the depressurization time span, at times where it is more appropriate so to not cause disturbances to the patient/user or other individuals around the patient/user.

Further embodiments are defined in the dependent claims. It should be emphasized that the term "comprises/comprising" when used in this specification is taken to specify the presence of stated features, integers, steps, or components. It does not preclude the presence or addition of one or more other features, integers, steps, components, or groups thereof.

These and other features and advantages of the disclosed technology will in the following be further clarified with reference to the embodiments described hereinafter.

### BRIEF DESCRIPTION OF THE DRAWINGS

The above aspects, features and advantages of the disclosed technology, will be more fully appreciated by reference to the following illustrative and non-limiting detailed description of example embodiments of the present disclosure, when taken in conjunction with the accompanying drawings, in which:
Fig. 1 is a schematic illustration of a wound treatment system in accordance with some embodiments.
Fig. 2 is a schematic illustration of a wound treatment system in accordance with some embodiments.
Fig. 3 is a schematic flowchart representation of a method for operating an apparatus for providing negative pressure to a wound site in accordance with some embodiments.
Fig. 4 is a schematic flowchart representation of a method for operating an apparatus for providing negative pressure to a wound site in accordance with some embodiments.
Fig. 5 is a schematic flowchart representation of a method for operating an apparatus for providing negative pressure to a wound site in accordance with some embodiments.

### DETAILED DESCRIPTION

The present disclosure will now be described in detail with reference to the accompanying drawings, in which some example embodiments of the disclosed technology are shown. The disclosed technology may, however, be embodied in other forms and should not be construed as limited to the disclosed example embodiments. The disclosed example embodiments are provided to fully convey the scope of the disclosed technology to the skilled person. Like reference characters refer to like elements throughout. Those skilled in the art will appreciate that the steps, services and functions explained herein may be implemented using individual hardware circuitry, using software functioning in conjunction with hardware circuitry such as a programmed microprocessor or general-purpose computer, using one or more Application Specific Integrated Circuits (ASICs), using one or more Field Programmable Gate Arrays (FPGA) and/or using one or more Digital Signal Processors (DSPs).

It will also be appreciated that when the present disclosure is described in terms of a method, it may also be embodied in an apparatus comprising one or more processors, one or more memories coupled to the one or more processors, where computer code is loaded to implement the method. For example, the one or more memories may store one or more computer programs that cause the apparatus to perform the steps, services and functions disclosed herein when executed by the one or more processors in some embodiments.

It is also to be understood that the terminology used herein is for purpose of describing particular embodiments only, and is not intended to be limiting. It should be noted that, as used in the specification and the appended claim, the articles "a", "an", "the", and "said" are intended to mean that there are one or more of the elements unless the context clearly dictates otherwise. Thus, for example, reference to "a unit" or "the unit" may refer to more than one unit in some contexts, and the like. Furthermore, the words "comprising", "including", "containing" do not exclude other elements or steps. It should be emphasized that the term "comprises/comprising" when used in this specification is taken to specify the presence of stated features, integers, steps, or components. It does not preclude the presence or addition of one or more other features, integers, steps, components, or groups thereof. The term "and/or" is to be interpreted as meaning "both" as well and each as an alternative. Similarly, "at least one of A and B" is to be interpreted as only A, only B, or both A and B.

It will also be understood that, although the term first, second, etc. may be used herein to describe various elements or features, these elements should not be limited by these terms. These terms are only used to distinguish one element from another. For example, a first signal could be termed a second signal, and, similarly, a second signal could be termed a first signal, without departing from the scope of the embodiments. The term "operatively connected" is in the context of the present disclosure to be understood as that the "operatively connected" entities or units can transmit and/or receive signals to and/or from each other.

As used herein, the term "in response to" may be construed to mean "when or "upon" or "if" depending on the context. Similarly, the phrase "if it is determined' or "when it is determined" or "in an instance of" may be construed to mean "upon determining or "in response to determining" or "upon detecting and identifying occurrence of an event" or "in response to detecting occurrence of an event" depending on the context. Accordingly, the phrase "if X equals Y" may be construed as "when X equals Y", "when it is determined that X equals Y", "in response to X being equal to Y", or "in response to detecting/determining that X equals Y" depending on the context.

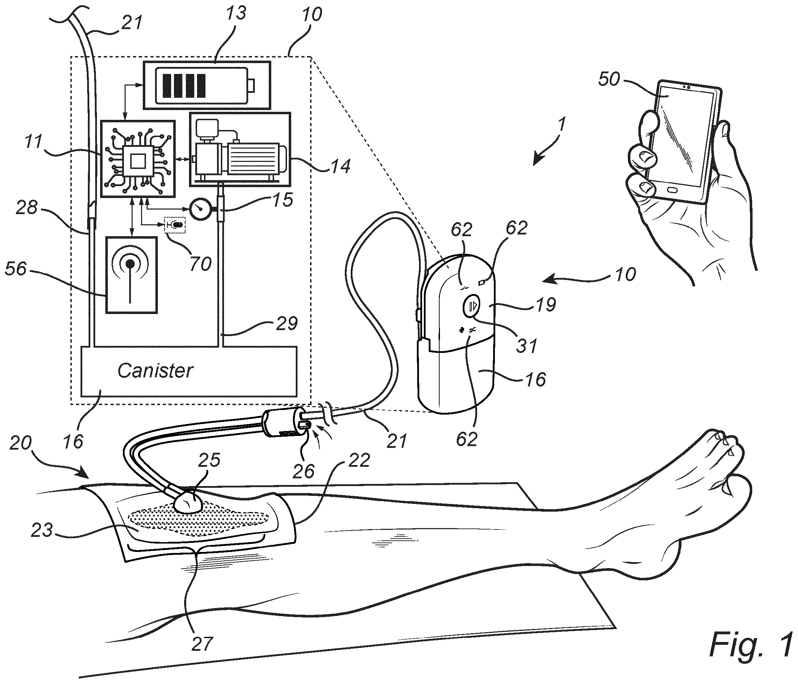

Turning now to the drawings, and to Fig. 1 in particular, there is schematically illustrated a wound treatment system 1 (sometimes simply referred to as "the system" 1) in accordance with some embodiments. The depicted wound treatment system 1 may be referred to as an "NPWT system" 1, "reduced pressure wound treatment system" 1, or "negative pressure wound treatment system" 1. The wound treatment system 1 comprises an apparatus 10 for providing reduced pressure to a tissue site 27 (or otherwise referred to as a wound site 27).

The wound treatment system 1 further comprises a wound cover 22 that is adapted to create a sealed space 23 defined in part by a wound surface, such as at the skin of a user/person, at or around a wound of the user/person. Further, the apparatus 10 (may also be referred to as an NPWT device 10) is fluidly connected to the wound cover 22 using e.g. a tubing 21. The tubing 21 may be of any suitable flexible tubing fabricated from elastomeric or polymeric materials. The tubing 21 may connect to the wound dressing 20, or more specifically to the wound cover 22 of the wound dressing 20, via a suitable connector 25. The connector 25 may be adhered or otherwise attached to the wound cover 22. In particular, the connector 25 may be attached around an opening (not shown) formed in the wound cover 22. The tubing 21 may comprise one, two, or more individual tubes.

The expression "wound cover" as used herein should be interpreted broadly as any wound site member, e.g. it can be a film sealed around a periphery of a wound site 27, where a wound filler may be used to fill the wound volume prior to the application of such wound cover. Wound cover may also refer to a backing layer of a wound dressing 20 comprising an additional layer(s) such as for example an absorbent layer and/or a spacer layer.

The apparatus 10 comprises a source of negative pressure 14 configured to provide negative pressure (i.e. sub-atmospheric pressure) via a fluid flow path from an inlet of the source of negative pressure 14 to the wound dressing 20, or more specifically to provide negative pressure under a wound cover. In some embodiments, the source of negative pressure 14 comprises a negative pressure pump that together with a motor is adapted for establishing a negative pressure when the source of negative pressure 14 is operating, i.e. when it is in an active state or simply "active". The source of negative pressure may comprise any type of pump and motor that are biocompatible or otherwise suitable for use in an NPWT setting and capable of maintaining or drawing adequate and therapeutic vacuum levels. Preferably, the negative pressure level to be achieved is in a range between about -20 mmHg and about -300 mmHg. In some embodiments, a negative pressure range between about -80 mmHg and about - 140 mmHg is used. In some embodiments, a negative pressure range between about -115 mmHg and about -135 mmHg is used. In some embodiments, the negative pressure pump is a diaphragm pump, a peristaltic pump, piezoelectric pump or the like, in which a motor causes the moving parts to draw fluid from the wound site 27. The term "pressure level" may also be referred to as "pressure value" or simply "pressure".

In the context of the present disclosure, it should be understood that the expressions "negative pressure", "sub-atmospheric pressure", "reduced pressure", or even "vacuum", as used interchangeably herein, generally refer to a pressure less than a local ambient pressure, such as the ambient pressure in a local environment external to a sealed therapeutic environment provided by a wound cover 22. In many cases, the local ambient pressure may also be the atmospheric pressure at which a patient is located. Thus, the "negative pressure" may be understood as a pressure difference between the ambient pressure and the pressure under the wound cover, where ambient pressure is generally set as 0 mmHg. Unless otherwise indicated, values of pressure stated herein are gauge pressures. Similarly, references to increases in negative pressure typically refer to a decrease in (absolute) pressure, while decreases in negative pressure typically refer to an increase in (absolute) pressure.

Further, in some embodiments the apparatus 10 comprises a canister 16 fluidly connected to the negative pressure source 14. The canister 16 may be formed from e.g. moulded plastic or the like, and possibly be a detachable component of the apparatus 10. Moreover, the canister 16 may further be at least partly transparent/translucent to permit viewing into the interior of the canister 16 to assist the user in determining the remaining capacity of the canister 16. As used herein, the term "fluidly connected" should be interpreted broadly and may comprise e.g. any form of tubing, conduits, or channels providing a fluid connection/communication between the canister 16 and the negative pressure source 14 and the dressing 20.

In some embodiments, the canister 16 comprises an inlet port 28 for allowing connection to the tubing 21. The inlet port 28 may also be formed elsewhere at the apparatus 10, however still fluidly connected to the canister 16. The connection between the inlet port 28 and the tubing 21 is a sealed connection, thus ensuring that no leakage is formed at the inlet port 28 during normal operation of the apparatus 10. The tubing 21 is preferably detachably connected to the inlet port 28 through conventional means including a friction fit, bayonet coupling, snap fit, barbed connector, or the like. The inlet port 28 may be moulded/formed from the same material and/or at the same time as forming the canister 16. A similar sealed connection (e.g. using a flange insulation/"O-ring") is formed between the canister 16 (at the outlet port 29) and the source of negative pressure 14. The canister 16 may form a part of the first fluid flow path.

In some embodiments, the apparatus comprises a housing 19 enclosing the negative pressure source 14. The canister 16 may be detachably connected to a housing 19 comprising the negative pressure source 14, whereby e.g. a full canister may be removed and replaced with an empty (new) canister. In such embodiments it may be desirable to provide e.g. the canister 16 and the housing 19 with some form of engagement means for securing the canister 16 to the housing such that the canister 16 is not unintentionally removed from the housing 19. The engagement means may in one embodiment comprise a pair of flexible protrusions extending from the canister 16 and adapted to engage with e.g. corresponding locking grooves provided at the housing 19.

However, in some embodiments, the wound treatment system 1 is a canister-less wound treatment system (not shown), where the wound exudate is collected in an absorbent layer, or the like, of the wound dressing 20. In such embodiments, the fluid flow path from the wound dressing 20 to the negative pressure source 14 is provided with one or more suitable filters (not shown) that are adapted to allow gas flow from the wound site 27 to the source of negative pressure 14 and block the flow of liquids from the wound site 27 to the source of negative pressure 14, as readily understood by the person skilled in the art.

The apparatus 10 further comprises control circuitry 11 (may also be referred to as "a control unit", "a controller", or the like) operatively connected to the power supply circuitry 13 and the source of negative pressure 14. The control circuitry 11 may for example be connected to the power supply circuitry 13 and the source of negative 14 by suitable wiring. The control circuitry 11 is configured to control operation of the source of negative pressure 14. The control circuitry 11 may comprise a microprocessor, microcontroller, programmable digital signal processor or another programmable device. The control circuitry 11 may also, or instead, include an application specific integrated circuit (ASIC), a programmable gate array or programmable array logic, a programmable logic device, or a digital signal processor. Where the control circuitry 11 includes a programmable device such as the microprocessor, microcontroller or programmable digital signal processor mentioned above, the control circuitry 11 may further include computer executable code that controls operation of the programmable device. Thus, in some embodiments, the control circuitry 11 comprises one or more processors and a memory, where the memory contains instructions executable by the processor whereby the apparatus 10 is operative to execute any one of the method steps or functions disclosed herein.

Furthermore, the apparatus 10 comprises power supply circuitry 13 comprising a power source, such as e.g. a battery for powering the apparatus 10. The battery may preferably be of the rechargeable type but may alternatively be arranged to be disposable and thus to be changed once discharged. A specifically adapted battery pack may be used in relation to some embodiments. The apparatus 10 may be of single-use type (allowing use during one single treatment time duration) or multiple-use type (allowing use during several different treatment sessions).

The power supply circuitry 13 may further comprise suitable components to regulate the supply voltage that is provided to various components of the apparatus 10, such as to the source of negative pressure 14, the general control circuitry 11, and so forth. For example, the power supply circuitry 13 may comprise one or more voltage regulators/converters (e.g., boost regulators or buck-boost regulators). However, in some embodiments, the voltage regulators/converters are provided as a part of the control circuitry 11 depending on requirements. However, in cases where the voltage regulators/converters are provided as a separate component to the control circuitry 11, they are still operatively connected to the control circuitry 11 and therefore controlled by the control circuitry 11.

Furthermore, in some embodiments, the apparatus 10 comprises one or more pressure sensors 15 arranged in fluid connection with the inlet of the source of negative pressure 14, the canister 16, and/or the sealed space 23. In the depicted embodiment of Fig. 1, the apparatus 10 comprises a pressure sensor 15a arranged in a fluid flow path between the canister 16 and the source of negative pressure 14. However, as readily understood by a person skilled in the art, the pressure sensor 15a may be located at any other suitable location to sense or detect a pressure within the fluid flow path between the source of negative pressure 14 and the wound site. For example, the pressure sensor 15a may be arranged within the canister or under the wound cover 22.

During use of the apparatus 10, the wound cover 22 is arranged at a wound site 27 of the user/patient, forming the sealed space 23. A tubing assembly is provided with a first tubing 21 to fluidly connect the wound cover 22 to the inlet port 28 of the apparatus 10. The apparatus 10 is then activated, e.g. by a user pressing a start/pause button 31 of a user-interface of the apparatus 10 that activates the source of negative pressure 14. Alternatively, activation may be initiated via an external device 50 connected to the system 1. The apparatus 10 may for example comprise a suitable communication interface 56 to wirelessly connect to the external device 50.

When activated, the source of negative pressure 14 will start to evacuate air through the canister 16, the inlet port 28, the tubing 21 and the sealed space 23 formed by the wound cover 22. Accordingly, a negative pressure will be created within the sealed space 23. This initial evacuation of air following a start-up of the apparatus 10 may be referred to as "depressurization". The initial evacuation of air (i.e., "depressurization") may continue until reaching a set pressure value (e.g., -125 mmHg). In some embodiments, the control circuitry 11 is configured to control the source of negative pressure 14 so to establish and maintain a negative pressure level (as measured by the pressure sensor 15) within a negative pressure range. The negative pressure range may for example be -20 mmHg to -300 mmHg, - 80 to -180 mmHg, -100 to -150 mmHg, -110 to -140 mmHg, -115 to -135 mmHg, or any suitable subrange thereof (e.g., -80 mmHg to -100 mmHg). The negative pressure range may for example be selected in view of the type of wound and/or the patient.

In case a liquid has been formed at the wound site 27, this liquid from the wound site 27 may at least partly be "drawn" from the wound site, through the tubing 21, the inlet port 28 and into the canister 16. The amount of liquid (possibly defined as exudate) that is drawn from the wound and collected in the canister 16 will depend on the type of wound that is being treated, the duration of the treatment, as well as the type of wound dressing 20 used. For example, in case an absorbent dressing is used, the liquid may be absorbed and collected both in the canister 16 and the wound dressing 20, whereas if a dressing with no absorption capacity or little absorption capacity is used, most or all of the liquid from the wound site 27 may be collected in the canister 16. A suitable filter member (not shown) is generally arranged in proximity to the outlet port 29 of the canister 16 to ensure that no liquid is allowed to pass to the source of negative pressure 14 from the canister 16.

In some embodiments, the wound treatment system 1 further comprise an air inlet port 26 or a controlled leakage port 26 that provides a small and controlled inflow of air (as indicated by the arrows) under the wound cover 22. The air inlet port 26 may for example be provided via a secondary lumen extending from the connector 25 as schematically illustrated. However, in some embodiments the air inlet port 26 may additionally or alternatively be provided at the connector 25 and/or along any suitable location of the tubing 21.

Accordingly, the wound treatment system 1 may in some embodiments be arranged to ensure that a small controlled leakage is present, ensuring that a flow from the sealed space 23 may be achieved (under the assumption that there is no general blocking e.g. within the tubing 21). Additionally, during use of the wound treatment system 1 some leakage may be expected in relation to the wound cover 22. In some embodiments, the air inlet port 26 includes means for supplying/providing air to the wound site 27 at a predetermined supply rate, the rate being from 2 to 7 ml/min, preferably from 3 to 5 ml/min at a predetermined level of negative pressure. The predetermined level of negative pressure may be a value within the range from -80 to -180 mmHg, preferably from -100 to -150 mmHg, more preferably from -110 to -140 mmHg, from -115 to -135 mmHg, or -120 to -135 mmHg. The means for providing air to the wound site 27 (i.e. under the wound cover 22) at a predetermined supply rate may for example be in the form of an air filter (not shown).

In some embodiments, the air filter comprises a hydrophobic and porous material, where the size of the pores is within the range of from 2 to 20 µm, preferably in the range of from 5 to 12 µm. The pore size of the filter is measured in a non-compressed state. In some embodiments, the air filter comprises polyethylene or sintered polyethylene.

As mentioned, the apparatus 10 may comprise a communication interface 56. The communication interface 56 may comprise suitable components (e.g. transceivers, antennas, filters, power amplifiers, etc.) with suitable communication protocols (e.g., Wi-Fi, Bluetooth, Bluetooth Low Energy, Zigbee, or the like) in order to establish a connection with an external device 50 and to transmit and receive wireless signals to and from the external device 50. The communication interface 56 is operatively connected to the control circuitry 11. The communication interface 56 may be configured to transmit and receive wireless signals to and from an external handheld device 50. In more detail, the communication interface 56 comprises one or more transceivers and one or more antennas connected to the one or more transceivers. The one or more transceivers are accordingly configured to transmit signals to an external device 50 via the one or more antennas and to receive signals from an external device 50 via the one or more antennas. For example, the communications interface 56 may include a Wi-Fi transceiver for communicating via a wireless communications network, cellular/mobile phone communications transceiver. In another example, the communication interface 56 may include a short-range wireless transceiver (e.g., a Bluetooth wireless transceiver, etc.) for communicating via a short-range wireless communication network. The external device may be an external handheld device 50, such as a mobile phone (e.g. a smart phone), that is operatively connected to the apparatus 10 via a suitable wireless connection.

Still further, the apparatus 10 may comprise one or more Light Emitting Diodes (LEDs) 62. The one or more LEDs 62 may be considered to form a User Interface (Ul) together with the button 31 arranged on the housing 19. The LEDs 62 may for example be arranged on the housing 19 of the apparatus 10. The LEDs 62 may be arranged under partly transparent portions of the housing to indicate various operating conditions of the apparatus 10. In more detail, the LEDs may be used to indicate a State of Charge (SoC) or available capacity of the power source 13, presence of a blockage condition, and/or a presence of a leakage condition. The aforementioned transparent portions may accordingly be formed as suitable elements/icons indicating the various operating conditions of the apparatus 10 (e.g., a battery-shaped element/icon for indicating battery capacity). Moreover, each LED may be arranged to emit light of different colours (e.g., green, yellow, red) to provide more granularity in the indications. For example, a green battery icon may indicate high battery capacity (e.g., SoC > 50%), a yellow battery icon may indicate a moderate battery capacity (e.g., 50% > SoC > 10%), while a red battery icon may indicate a low battery capacity (e.g., SoC < 10%). The apparatus 10 may comprise further LEDs than those indicated in the drawings. For example, the apparatus 10 may comprise an LED associated with the button 31 in order to indicate an active state of the apparatus 10.

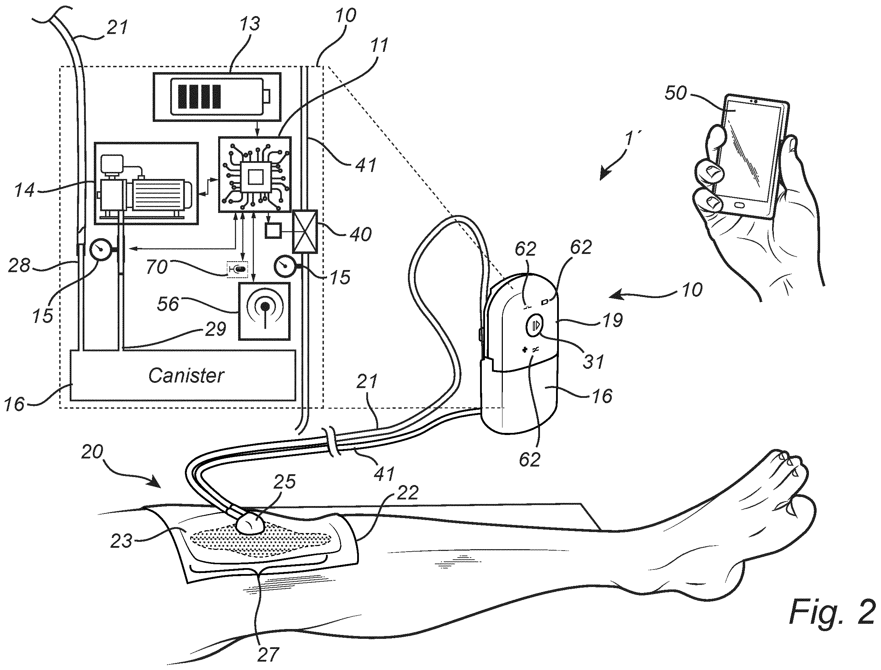

Fig. 2 depicts a wound treatment system 1' in accordance with some embodiments. Many functions, features and components of the wound treatment system 1' depicted in Fig. 2 are the same as or analogous to the wound treatment system 1 depicted in Fig. 1 and will for the sake of brevity and conciseness not be repeated. Instead, the focus will be on the differentiating functions, features, or components between the two wound treatment systems. Thus, as the person skilled in the art readily understands, unless specifically indicated, the foregoing discussion related to the components of the wound treatment system 1 with reference to Fig. 1 is applicable for the corresponding components depicted in Fig. 2 as indicated by the reference numerals.

In general, the wound treatment system 1 depicted in Fig. 1 differs from the wound treatment system 1' depicted in Fig. 2 in that the former is often referred to as a "single-lumen" NPWT system 1 while the latter is often referred to as a "dual-lumen" NPWT system 1'. Accordingly, while the wound treatment system 1 depicted in Fig. 1 has a "controlled inflow of air" via leakage (also referred to as a controlled leakage), the wound treatment system 1' depicted in Fig. 2 does not have a controlled leakage, but instead explicitly controls or regulates the air/fluid supply to the wound site 27 by opening and closing the valve 40.

Therefore, in contrast to the wound treatment system 1 depicted in Fig. 1, the wound treatment system 1' of Fig. 2 comprises a second fluid flow path that fluidly connects the sealed space 23 created by the wound cover 22 to the ambient atmosphere or a "fluid reservoir" (not shown) via an electronically controllable valve 40. The second fluid flow path (may also be referred to as "air lumen") is defined by a second tubing 41 that may be of any suitable flexible tubing fabricated from elastomeric or polymeric materials. Here, the fluid flow path from the inlet of the source of negative pressure 14 to the wound dressing 20 may be construed as a "first" fluid flow path or "exudate lumen".

Here, the control circuitry 11 is operatively connected to the electronically controllable valve 40, and the control circuitry 11 is further configured to control an operation of the electronically controllable vale 40 so to release negative pressure from the wound site via the second fluid flow path (or "air lumen") 41. In other words, the control circuitry 11 is configured to open the electronically controllable valve 40 so to introduce fluid (e.g. air) to the wound site 27 when there is a sub-atmospheric pressure under the wound cover 22. This operation may also be referred to as "flushing".

Since the wound treatment system 1' depicted in Fig. 2 does not have any "controlled leakage flow", there is a risk that the apparatus 10 would not be able to draw any, or at least a sub-optimal amount of "exudate" from the wound site 27 to the canister 16 once negative pressure has been established under the wound cover 22 due to lack of airflow through the system 1'. Therefore, in order to be able to draw desired amounts of wound exudate from the wound site, a fluid, such as e.g. air from the ambient atmosphere, may be controllably introduced at defined time intervals and/or in response to pressure measurements by opening the electronically controllable valve 40. Once enough fluid has been introduced (e.g. in response to a negative pressure within the first and/or second fluid flow path reaching a lower threshold), the control circuitry 11 is configured to close the electronically controllable valve 40 and to activate the source of negative pressure 14.

The ability to controllably "flush" the system 1' by injecting air via an electronically controllable valve 40 may provide the advantage of longer pressure regulation cycles, which reduces the on-time for the source of negative pressure 14 and therefore reduces the energy consumption of the apparatus 10.

As before, the apparatus 10 of the wound treatment system 1' may comprise one or more pressure sensors 15a arranged in fluid connection with the inlet of the source of negative pressure 14, the canister 16, and/or the sealed space 23. However, the apparatus 10 may alternatively or additionally comprise a pressure sensor 15b arranged in fluid connection with the second fluid flow path (i.e., "air lumen"). The pressure sensor 15b (i.e., "air lumen pressure sensor") is preferably arranged to measure a pressure level in the second fluid flow path downstream of the electronically controllable value 40 (i.e., between the valve 40 and the wound site 27). In the depicted embodiment of Fig. 2, the apparatus comprises two pressure sensors 15a, 15b, a first pressure sensor 15a configured to measure a pressure level within the first fluid flow path, and a second pressure sensor 15b configured to measure a pressure level within the second fluid flow path. However, the apparatus 10 may comprise a single pressure sensor arranged in any one of the suitable locations mentioned in the foregoing.

The apparatus 10 may further comprise an air filter (not shown) arranged in the second fluid flow path in order to limit the airflow when the electronically controllable valve 40 is open. In some embodiments, the air filter comprises a hydrophobic and porous material, where the size of the pores is configured to allow for a flow in the range of 50-120 ml/s, such as 60-100 ml/s when the valve 40 is opened after negative pressure has been established under the wound cover 20. The pore size of the filter is preferably measured in a non-compressed state. In some embodiments, the air filter comprises polyethylene or sintered polyethylene. The air filter may also serve to reduce the risk of contaminants entering the wound site 27 when the valve 40 is opened (i.e., during "flushing"). The air filter may be arranged in the tube 41 that at least partly defines the second fluid flow path. The air filter may be arranged upstream of the valve 40 (i.e., between the valve 40 and the inlet of ambient air, or downstream of the valve 40 such as between the valve and the air lumen pressure sensor 15.

Regardless of the wound treatment system 1, 1' being single lumen (Fig. 1) or dual lumen (Fig. 2), the control circuitry 11 is configured to in response to receiving an indication that a noise level of ambient noise is above a noise level threshold, set the supply voltage to the source of negative pressure to a first voltage value above a nominal voltage value. The term "first voltage value" will generally be referred to as a "boost voltage" in the following in order to improve readability.

The term "nominal voltage" refers to the standard or designated voltage value at which the source of negative pressure 14 is designed to operate under normal conditions. As used herein, the nominal voltage value may be a predefined voltage value to be provided to the source of negative pressure 14 by the power supply circuitry 13 when the pressure change rate as detected by the pressure sensor 15a, 15b is above the pressure change rate threshold. The term "boost voltage" (or "first voltage value") refers to a temporary increase in voltage above the nominal level that is used under certain conditions or circumstances as exemplified herein.

In some embodiments, the apparatus 10 comprises a microphone 70 configured to monitor the noise level of ambient noise, and the control circuitry 11 is operatively connected to the microphone 70. In more detail, the housing may comprise a perforation or grille and the microphone 80 may be arranged in close proximity to this perforation or grille in order to capture sound without obstruction. Moreover, the apparatus 10 may further include soundproof materials or enclosures around internal components (e.g., the source of negative pressure 10) to minimize interference from the apparatus 10 itself. The microphone 70 may be an omnidirectional microphone. The microphone 70 may be mounted on a soft or flexible material to reduce noise from handling the apparatus 10 or from movement of the apparatus 10. The microphone 70 may for example be an electret condenser microphone.

The noise level threshold may for example be a fixed value, such as for example a value within the range of 40 dBA to 60 DBA, such as 40 dBA, 45 dBA, 50 dBA, 55 dBA or 60 dBA. Moreover, the noise level threshold may be set based on the noise level of the source of negative pressure 14 when it is operating with a nominal supply voltage. For example, if the noise level of the source of negative pressure is 45 dBA when it is operating with a nominal supply voltage, the noise level threshold may be set to 45 dBA or 50 dBA. By setting the threshold in dependence of the noise level of the source of negative pressure 14 when it is operating with a nominal supply voltage, the increased noise level emitted by source of negative pressure 14 when its operated at a boosted supply voltage is less perceivable by the user/patient as the ambient noise may drown out some of the noise emitted by the apparatus 10.

However, in some embodiments, the noise level threshold may be set based on the noise level of the source of negative pressure 14 when it is operating with a boosted supply voltage. For example, if the noise level of the source of negative pressure is 55 dBA when it is operating with a boosted supply voltage, the noise level threshold may be set to 55 dBA or 60 dBA. By setting the threshold in dependence of the noise level of the source of negative pressure 14 when it is operating with a boosted supply voltage, the increased noise level emitted by source of negative pressure 14 when its operated at a boosted supply voltage is even less perceivable by the user/patient as the ambient noise is louder than the noise emitted by the apparatus 10.

Boosting the supply voltage to the source of negative pressure 14 at times with elevated ambient noise (above the noise level threshold), the drawback of increased noise being emitted from the apparatus 10 is at least partly mitigated while the advantage of more time-efficient pump operation is still achievable. Moreover, by having the boosted supply voltage triggering on ambient noise levels aids to ensure that the boosted supply voltage, which will inevitably cause the apparatus 10 to be louder than the nominal supply voltage, is not applied at inappropriate moments such as when the user/patient is sleeping or resting in a quiet environment, thereby improving the user experience.

Moreover, in some embodiments, the control circuitry 11 is configured to, in response to receiving an indication that the noise level of ambient noise is below the noise level threshold, set the supply voltage to the source of negative pressure 41 to the nominal voltage value.

As readily understood by the skilled reader, the setting of the supply voltage to the source of negative pressure 14 may be immediately preceded or superseded by an activation of the source of negative pressure 14, unless the source of negative pressure is already active and the setting of the supply voltage is a change of supply voltage, in which case the setting of the supply voltage obviously supersedes the activation. Alternatively, the setting of the supply voltage to the source of negative pressure 14 activates the source of negative pressure 14. In other words, the setting of the supply voltage causes the activation of the source of negative pressure 14, meaning that the two (setting and activating) occur substantially at the same time. As before, this does not really apply if the source of negative pressure 14 is already active and the setting of the supply voltage is a change of supply voltage.

Further, in some embodiments, the noise level threshold is a range of noise level values, and wherein the noise level threshold for setting the supply voltage to the first voltage value is an upper value of the range of noise level values and the noise level threshold for setting the supply voltage to the nominal voltage value is a lower value of the range of noise level values. In other words, a noise level hysteresis band may be applied to define the noise level threshold. In more detail, if for example the noise level threshold is defined as a noise level range between 40 dBA and 50 dBA, then the detected noise level has to exceed 50 dBA in order to provide the boosted supply voltage to the source of negative pressure 14 and fall below 40 dBA in order to switch the supply voltage to the nominal voltage value. Accordingly, if the detected noise level goes from 55 dBA to 45 dBA, it will still be the boost voltage value that is used as the supply voltage, and similarly if the noise level goes from 30 dBA to 45 dBA, it will still be the nominal voltage value that is used as the supply voltage.

Moreover, in a scenario where the source of negative pressure 14 has been off/inactive for a while, and the source of negative pressure 14 is to be started and the noise level is within this "range of noise level values", the control circuitry may be configured to apply the most recently set supply voltage. Assuming that the noise level never passed any of the end-values and that the apparatus is continuously monitoring the noise level. However, if the apparatus is only monitoring the noise level when the source of negative pressure 14 is to be activated and/or while it is active, and the noise level is within this "range of noise level values", the control circuitry 11 may be configured to use the most recently set supply voltage.

An advantage of using a hysteresis band as the noise level threshold is that rapid switching back and forth between two different supply voltage values may be avoided and reduce the risk of damage to the electrical components of the apparatus 10, as compared to using a single fixed threshold value and the detected noise level is at or around the single fixed threshold value. Rapidly switching supply voltages to the source of negative pressure 14 may be associated with different negative effects depending on the type of pump and motor that is used for the source of negative pressure 14. For example, for piezoelectric motors, rapid switching of the supply voltage could cause reduced efficiency, material fatigue, and irregular movement. For diaphragm pumps and the associated reciprocating motor, rapid switching of the supply voltage could cause erratic pumping, mechanical stress on the diaphragm, and reduced motor life. For peristaltic pumps and the associated DC or stepper motor, rapid switching of the supply voltage could cause reduced pump and motor life, higher power consumption, overheating, and pulsations or pressure surges. Moreover, rapid switching between supply voltages may cause damage to the power supply itself due to spikes or fluctuations in the current drawn from the power supply, and/or damage sensitive components like capacitors, diodes, and transistors of associated control circuitry.

Moreover, in some embodiments, the control circuitry 11 is further configured to, in response to a pressure level as detected by the pressure sensor 15a, 15b being below a pressure level threshold and the noise level of ambient noise being above the noise level threshold, set the supply voltage to the source of negative pressure 14 to the nominal voltage value. In other words, the apparatus 10 may be configured to not only check that the noise level is above some threshold before triggering the boost voltage supply, but to also check that the pressure level is above some set pressure level threshold. In reference to "negative pressure" rather than "pressure", the control circuitry 11 may be configured to, in response to a negative pressure level as detected by the pressure sensor 15a, 15b being above a negative pressure level threshold and the noise level of ambient noise being above the noise level threshold, set the supply voltage to the source of negative pressure 14 to the nominal voltage value.

Moreover, a purpose of using the detected pressure as another condition for controlling the supply voltage to the source of negative pressure is that the control circuitry 11 may be configured to reduce supply voltage to nominal value when approaching a target pressure regardless of the noise level. Thereby, the risk of overshooting the target pressure and causing discomfort to the user/patient may be reduced. Accordingly, in some embodiments, the pressure level threshold is higher than the target pressure, or analogously, the negative pressure level threshold is lower than a target negative pressure. For example, if the target pressure is -125 mmHg, the pressure level threshold may be set to -120 mmHg, -115 mmHg, or -110 mmHg.

Additionally, by utilizing a pressure level threshold as an additional condition for triggering the boost voltage, one can ensure that the boost voltage is supplied only when the apparatus 10 is in the initial depressurization state and not in the therapy regulation state. For example, during the initial depressurization state the pressure level at the wound site is brought from 0 mmHg to a target pressure (e.g., -125 mmHg), while during the therapy regulation state, the pressure level at the wound site may be cycled between some lower pressure value and upper pressure value (e.g., between -120 mmHg and -130 mmHg). Thus, by setting the pressure level threshold to for example -115 mmHg, the boost voltage supply should not be applied while the apparatus 10 is operating in the therapy regulation state. The pressure level threshold may also be set as a percentage of the target pressure, such as 75%, 80%, 85%, or 90% of the target pressure. For example, if the target pressure is -125 mmHg, the pressure level threshold may be set to -112,5 mmHg (i.e., 90% of the target pressure).

An advantage of only supplying the boost voltage during initial depressurization and not during the therapy regulation is that there is a trade-off between time-efficiency and noise. As the range of pressure levels that the apparatus 10 regulates between during pressure regulation is generally much smaller than during initial depressurization the gain in time-efficiency is limited while the increased noise may be more disadvantageous as it can induce a startling effect to the patient/user since the source of negative pressure is automatically activated. In contrast, the gain in time-efficiency is relatively high during the initial depressurization and the source of negative pressure 10 is activated by the Healthcare Professional (HCP) or patient/user which reduces the risk of the patient/user being startled by the noise.

In some embodiments, the control circuitry 11 is configured to, in transition from the first voltage value to the nominal voltage value, set the supply voltage to the nominal voltage value by gradually reducing the supply voltage from first voltage value to the nominal voltage value. In other words, in transition from the boost voltage value to the nominal voltage value, the setting of the supply voltage to the nominal voltage value comprises gradually reducing the supply voltage from boost voltage value to the nominal voltage value.

This gradual reduction in supply voltage from the boost voltage to the nominal voltage may be time-controlled or pressure-controlled. In more detail, the gradual reduction of the supply voltage may comprise gradually reducing the supply voltage over a set period of time. However, the gradual reduction of the supply voltage may alternatively comprise gradually reducing the supply voltage over a set range of pressure level values.

For example, the set period of time may be 5 seconds, and the boost voltage may be 5V while the nominal voltage may be 3V. Then the gradual reduction may accordingly comprise setting the voltage to 4,6V after 1 second, to 4,2V after 2 seconds, 3,8V after 3 seconds, 3,4V after 4 seconds, and 3V after 5 seconds. Similarly, using the same voltage values, a pressure-controlled reduction from -115 mmHg (pressure threshold for boost voltage) to -125 mmHg (target pressure) may comprise setting the voltage to 4,6V after reaching -117 mmHg, to 4,2V after reaching -119 mmHg, 3,8V after reaching -121 mmHg, 3,4V after reaching -123 mmHg, and 3,0V after reaching -125 mmHg. It should be noted that the time period, voltage values, and pressure values define merely one example configuration out of several suitable configurations to set the time-controlled or pressure-controlled reduction in supply voltage, and it need not necessarily be a linear reduction. For example, the set period of time may be a value in the range of 2-10 seconds, the boost and nominal voltages are dependent on the specifications for the source of negative pressure and may range from 2V to 10V, and the pressure values may depend on the set target pressure, the set pressure level threshold and the difference therebetween.

As mentioned, the apparatus 10 may comprise a communication interface 56 having at least one antenna and at least one transceiver operatively connected to the at least one antenna. Thus, in some embodiments, the control circuitry 11 is configured to receive a signal comprising an indication of the noise level of ambient noise from an external handheld device 50. Advantageously, the communication interface 56 may include a short-range wireless transceiver (e.g., a Bluetooth wireless transceiver, etc.) for communicating via a short-range wireless communication network. Thereby, the connected external device 50 is likely to be in relatively close proximity to the apparatus 10 and the sound level measurements of the external device 50 are more likely to represent the noise level of ambient noise in the surrounding environment of the apparatus 10.

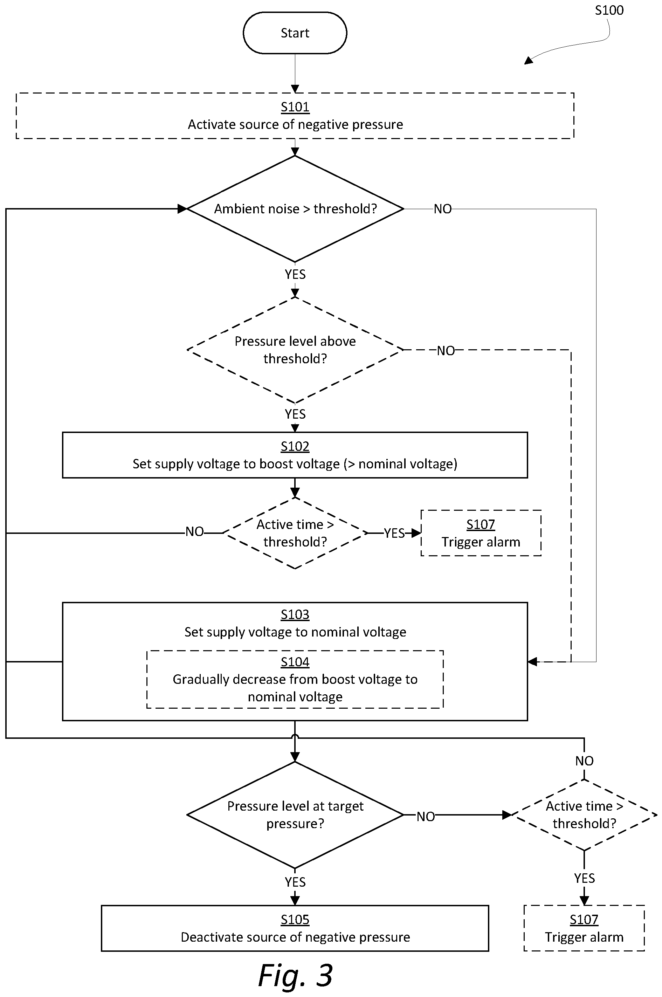

In the following, further functions and operations of the control circuitry 11 of the apparatus 10 will be discussed in with references the flowcharts depicted in Fig. 3 to Fig. 5. In particular, Figs. 3-5 are schematic flowchart representations of methods S100, S200, S300 for operating an apparatus 10 for providing negative pressure to a wound site 27 in accordance with some embodiments. Even though the methods S100, S200, S300 are depicted as separate processes in separate figures, the various functions may be combined without departing from the scope of the present invention as readily understood by the skilled person in the art. The flowcharts are mainly separated in order to ensure readability.

Thus, Fig. 3 is a flowchart representation of a method S100 for operating an apparatus 10 in accordance with some embodiments. The method S100 is preferably a computer-implemented method S100, performed by a processing system of the apparatus 10. The processing system may for example comprise one or more processors 11 and one or more memories coupled to the one or more processors 11, wherein the one or more memories store one or more programs that perform the steps, services and functions of the method S100 disclosed herein when executed by the one or more processors.

The method S100 may comprise activating S101 the source of negative pressure 14. Further, in response to receiving an indication that a noise level of ambient noise is above a noise level threshold, the method S100 comprises setting S102 the supply voltage to the source of negative pressure 14 to a first voltage value above a nominal voltage value. Thus, a check is performed to see whether the noise level as measured by the microphone 70 or as reported by the external device 50 is above the threshold. Then, if the noise level is above the threshold, the supply voltage to the source of negative pressure 14 is set S102 to a boost voltage (e.g., 5V) higher than the nominal voltage (e.g., 3V). However, if the noise level is below the threshold, the supply voltage to the source of negative pressure 14 is set S103 to the nominal voltage (e.g., 3V).

As before, the setting S102, S103 of the supply voltage to the source of negative pressure 14 may be preceded or superseded by the activation S101 of the source of negative pressure 14, unless the source of negative pressure is already active and the setting of the supply voltage is a change of supply voltage, in which case the setting S102, S103 of the supply voltage obviously supersedes the activation. Alternatively, the setting S102, S103 of the supply voltage to the source of negative pressure 14 activates S101 the source of negative pressure 14. In other words, the setting of the supply voltage causes the activation of the source of negative pressure 14, meaning that the two (setting S102, S103 and activating S101) occur substantially at the same time.

Furthermore, in some embodiments, the method S100 further comprises a voltage value check as another condition to set S102 the supply voltage to the boost voltage. In more detail, in response to the noise level being above the noise level threshold, the method S100 may comprise setting S102 the supply voltage to the source of negative pressure 14 to the boost voltage value while the pressure level as detected by the pressure sensor 15a, 15b is above a pressure level threshold. Thus, instead of only controlling the supply voltage based on the noise level, another condition related to the detected pressure level is added.

In other words, in response to the noise level being above the noise level threshold and the detected pressure level being above a pressure level threshold, the supply voltage to the source of negative pressure 14 may be set S102 to the boost voltage value. However, should the noise level be above the noise level threshold and the detected pressure level be below a pressure level threshold, the supply voltage to the source of negative pressure 14 may be set S103 to the nominal voltage value

Thus, while the noise level remains high (above threshold) and the detected pressure level is above the pressure level threshold, the source of negative pressure 14 is supplied with a boost voltage value. However, when the detected pressure level goes below the pressure level threshold, the supply voltage is switched from the boost voltage value to the nominal voltage value. The pressure level threshold may for example be set to a value above the target pressure level of the apparatus 10, such as e.g., 5 mmHg above the target pressure level, 10 mmHg above the target pressure level or 15 mmHg above the target pressure level. Thereby, the source of negative pressure 14 slows down when the detected pressure level approaches the target pressure level, and the risk of overshooting the target pressure level may be reduced. Once the detected pressure level reaches the target pressure level, the method S100 may comprise deactivating S105 the source of negative pressure 14.

Moreover, in some embodiments, in the transition from the boost voltage value to the nominal voltage value, the setting S103 of the supply voltage to the nominal voltage value comprises gradually reducing S104 the supply voltage from boost voltage value to the nominal voltage value. As mentioned in the foregoing, the gradual reduction S104 of the supply voltage may be time-controlled or pressure-controlled.

Further, the method S100 may comprise one or more checks of the active time of the source of negative pressure 14, i.e., checks to see how long the source of negative pressure 14 has been on or active. The active time of the source of negative pressure may be compared to a time threshold, and in response to the active time exceeding the time threshold, the method S100 may comprise triggering S107 an alarm. For example, if the source of negative pressure 14 has been running a long time (on-time is longer than threshold), but the pressure level as detected by the pressure sensor 15a, 15b still has not reached a target pressure level, this may be indicative of a presence of a leakage condition in a single or dual lumen therapy system 1, 1' or a blockage condition in a dual lumen system 1'.

A leakage condition may for example be due to improperly applied or detached wound cover 22 and/or leaky connections between the tubing and other components, and may be characterized as "unwanted leakage" in a single lumen therapy system 1 where there is a "controlled leakage". A blockage condition may be caused by an external impact such as a patient lying on a tube 21, 41 or a patient bending the tubing 41, 42. A blockage condition may also be caused by substances which are contained in the fluids sucked away from the wound (such as proteins) that may clog the tubing 21, 41. In order to more reliably detect the presence in the dual lumen system based on the active time of the source of negative pressure 14, the "air lumen pressure sensor" 15a output should preferably be used.

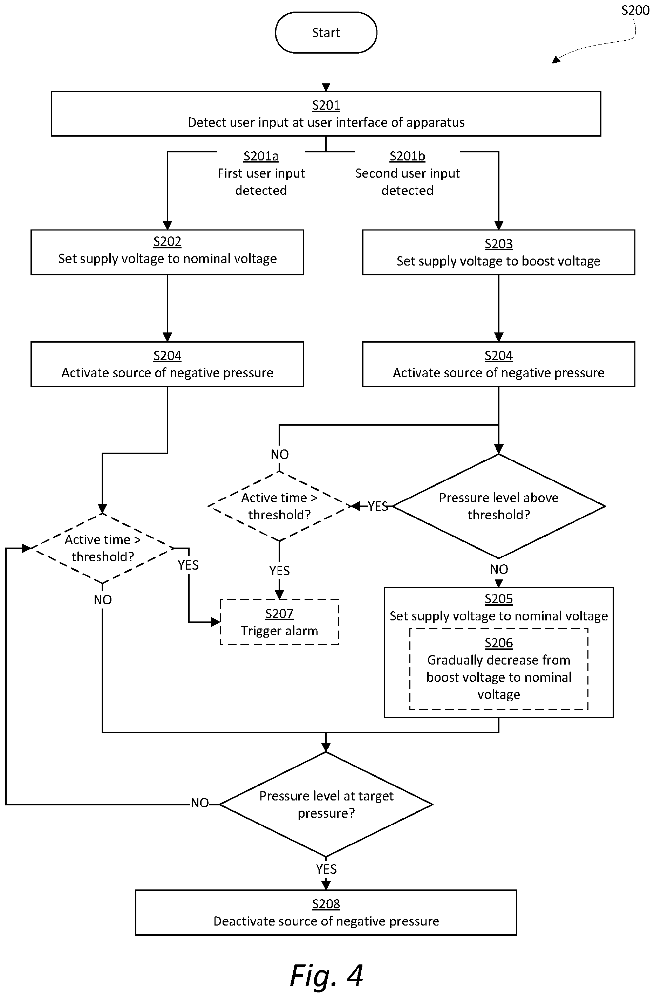

Fig. 4 is a schematic flowchart representation of a method S200 for operating an apparatus 10 in accordance with some embodiments. The method S200 is preferably a computer-implemented method S100, performed by a processing system of the apparatus 10. The processing system may for example comprise one or more processors 11 and one or more memories coupled to the one or more processors 11, wherein the one or more memories store one or more programs that perform the steps, services and functions of the method S200 disclosed herein when executed by the one or more processors.

In short, in the method S200, the "boost voltage" is applied in response to a particular user interaction, such as for example a long button press. In more detail, the control circuitry 11 of the apparatus may be configured to discern between different user interactions (e.g., short and long presses of the button 31 of the apparatus 10), and depending on which type of interaction is detected, the supply voltage to the source of negative pressure 14 is set to either the nominal voltage (e.g., 3V) or the boost voltage (e.g., 5V). An advantage of controlling the boost voltage in dependence of user interaction is that it allows a user/patient of the apparatus 10 or the HCP to decide when a more efficient, albeit louder, depressurization should be executed.

Accordingly, the method S200 may comprise detecting S201 a user input at a user interface of the apparatus 10. Further, in response to detecting S201a a first user input at the user interface of the apparatus 10, the method S200 comprises setting S202 the supply voltage to the source of negative pressure to a nominal voltage value and activating S204 the source of negative pressure 14. In other words, the control circuitry 11 is configured to, in response to detecting S201a a first user input at the user interface of the apparatus 10, set the supply voltage to the source of negative pressure to a nominal voltage value and activate the source of negative pressure 14.

Similarly, in response to detecting S201b a second user input at the user interface of the apparatus 10, the method S200 comprises setting S203 the supply voltage to the source of negative pressure 14 to the boost voltage value above the nominal voltage value and activating S204 the source of negative pressure 14. In other words, the control circuitry 11 is configured to, in response to detecting S201b a second user input at the user interface of the apparatus, set the supply voltage to the source of negative pressure 14 to a boost voltage value above the nominal voltage value and activate the source of negative pressure 14. Here, the second user input is different from the first user input. It should be noted that the order of the setting the supply voltage to the boost voltage and activating the source of negative pressure may be interchanged without departing from the scope.

For example, if the user interface of the apparatus 10 comprises a button 31, such as the start/pause button 31 in Fig. 1 and Fig. 2. The first user input may be a "short" button press (e.g., the button 31 is pressed down less than 1 second) while the second user input may be a "long" button press (e.g., the button is pressed down longer than 1 second). It should be noted that the time periods defining a "short" and "long" button press are mere examples and that different values may be used. In more general terms, the "short" button press involves a user pressing the button 21 for a shorter period of time than for the "long" button press. Moreover, in some embodiments the user interface of the apparatus 10 may comprise two different buttons, and the first and second user inputs may accordingly be pressing of the first and second button, respectively. Similarly, the user interface may comprise a touch screen and the first and second user inputs may be defined by different user-interactions on the touch screen, such as interactions on different parts of the touch screen, different types of interactions (press vs press and hold), and so forth.

Further, in some embodiments, and assuming that the source of negative pressure 14 is operating with the boost supply voltage, the method S200 comprises setting S205 the supply voltage to the source of negative pressure 14 to the nominal voltage value in response to the pressure level as detected by the pressure sensor 15a, 15b approaching or reaching a target pressure value. In other words, when the source of negative pressure is operating at the boost voltage, the pressure level as detected by the pressure sensor 15a, 15b is monitored, and upon approaching or reaching the target pressure value, the supply voltage is reduced to the nominal voltage value. The trigger to switch the supply voltage from the boost voltage to the nominal voltage may for example be in response to the pressure level as detected by the pressure sensor 15a, 15b reaching or falling below a pressure level threshold. The pressure level threshold may accordingly be the target pressure or a pressure value above the target pressure. In reference to the latter, the pressure level threshold may be 5 mmHg above the target pressure level, 10 mmHg above the target pressure level or 15 mmHg above the target pressure level. Thereby, the source of negative pressure 14 slows down when the detected pressure level approaches the target pressure level, and the risk of overshooting the target pressure level may be reduced. Once the detected pressure level reaches the target pressure level, the method S200 may comprise deactivating S208 the source of negative pressure 14.

Moreover, in some embodiments, in the transition from the boost voltage value to the nominal voltage value, the setting S205 of the supply voltage to the nominal voltage value comprises gradually reducing S206 the supply voltage from boost voltage value to the nominal voltage value. As mentioned in the foregoing, the gradual reduction S206 of the supply voltage may be time-controlled or pressure-controlled.

Further, the method S200 may comprise one or more checks of the active time of the source of negative pressure 14, i.e., checks to see how long the source of negative pressure 14 has been on or active. The active time of the source of negative pressure may be compared to a time threshold, and in response to the active time exceeding the time threshold, the method S200 may comprise triggering S207 an alarm. For example, if the source of negative pressure 14 has been running a long time (on-time is longer than threshold), but the pressure level as detected by the pressure sensor 15a, 15b still has not reached a target pressure level, this may be indicative of a presence of a leakage condition in a single or dual lumen therapy system 1, 1' or a blockage condition in a dual lumen system 1'.

Accordingly, in some embodiments, there is provided an apparatus 10 for providing negative pressure to a wound site. The apparatus comprises a source of negative pressure 14 configured to provide negative pressure via a first fluid flow path from an inlet of the source of negative pressure to a wound site 27, and power supply circuitry 13 comprising a power source. The power supply circuitry is configured to provide a supply voltage to the source of negative pressure 14. The apparatus 10 comprises a user interface having a user input device. The apparatus 10 further comprises control circuitry 11 operatively connected to the source of negative pressure 14 and the power supply circuitry 13. The control circuitry 11 is configured to in response to detecting a first user input at the user input device of the user interface of the apparatus 10, set the supply voltage to the source of negative pressure to a nominal voltage value and activate the source of negative pressure 14. Moreover, the control circuitry is configured to in response to detecting a second user input at the user interface of the apparatus different from the first user input, set the supply voltage to the source of negative pressure 14 to a first voltage value above the nominal voltage value and activate the source of negative pressure 14.

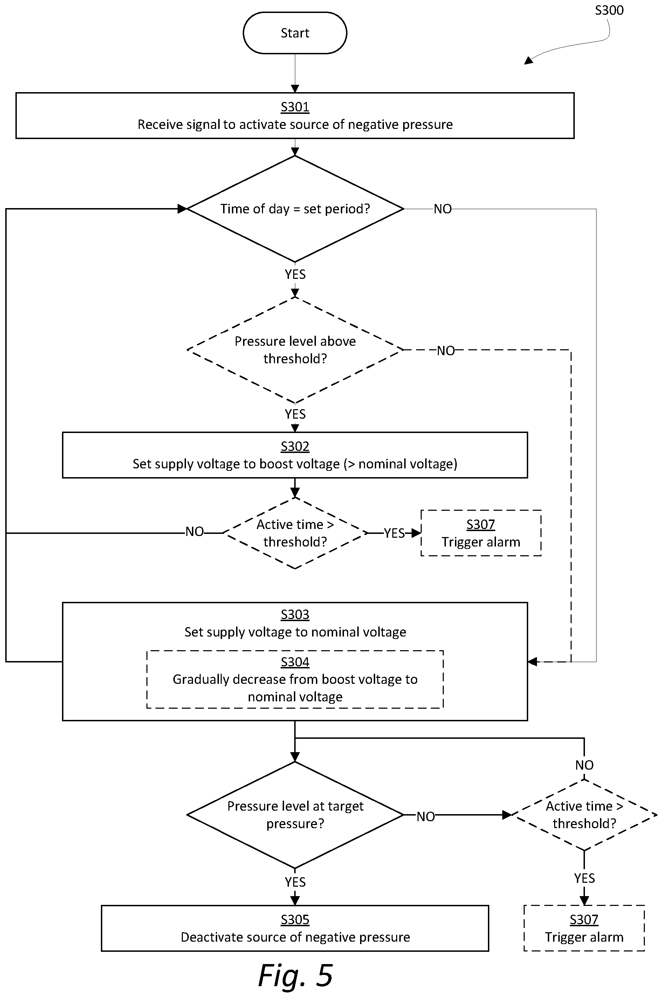

Fig. 5 is a schematic flowchart representation of a method S300 for operating an apparatus 10 in accordance with some embodiments. The method S300 is preferably a computer-implemented method S100, performed by a processing system of the apparatus 10. The processing system may for example comprise one or more processors 11 and one or more memories coupled to the one or more processors 11, wherein the one or more memories store one or more programs that perform the steps, services and functions of the method S300 disclosed herein when executed by the one or more processors.

In short, in the method S300, the "boost voltage" is applied in dependence of a specific time of the day. The apparatus 10 may obtained the information about a current time of day using an internal Real-Time Clock (RTC) or via data received from an external device 50. For example, the apparatus 10 may be adapted to set the supply voltage of the source of negative pressure 14 to the boost voltage value during "active" parts of the day, such as during day-time or specific subperiods thereof. Similarly, the apparatus may be adapted to set the supply voltage of the source of negative pressure 14 to the nominal voltage value during "inactive" parts of the day, such as during night-time or specific subperiods thereof.

Accordingly, the method S300 may comprise receiving S301 a signal to activate the source of negative pressure 14. In more detail, the signal to activate the source of negative pressure 14 may be generated in response to a user interaction (e.g., a user activating the apparatus 10), a pressure value reaching or falling below some threshold, a counter reaching a limit, or similar. In other words, the signal may be an internal signal in the control circuitry 11 that is triggered upon the occurrence of a specific event.

Further, in response to detecting that a current time of day corresponds to a set period of the day, the method S300 comprises setting S302 a supply voltage level to the source of negative pressure 14 to a boost voltage value that is higher than a nominal voltage value and activating the source of negative pressure 14. In other words, the control circuitry 11 is configured to set a supply voltage level to the source of negative pressure 14 to a boost voltage value that is higher than a nominal voltage value and activate the source of negative pressure 14. Analogously, in response to detecting that a current time of day does not correspond to the set period of the day, the method S300 comprises setting S303 a supply voltage level to the source of negative pressure 14 to a nominal voltage value and activating the source of negative pressure 14.

It should be noted that the order of the setting of the supply voltage and the activating the source of negative pressure may be interchanged without departing from the scope. The set period of the day may for example be one or more predefined periods or hours of the day such as for example between 10:00 and 18:00 or between 08:00 and 20:00. Moreover, the set period of the day may be individually configured and stored in each individual apparatus 10 depending on user-preferences.

As before, the time of day may be one out of multiple conditions that need to be fulfilled for the apparatus 10 to utilize a boosted voltage as the supply voltage for running the source of negative pressure 14. For example, another condition may be that the current pressure level detected by the pressure sensor 15a, 15b. Thus, the method S300 may comprise, in response to detecting that a current time of day corresponds to a set period of the day, setting S302 a supply voltage level to the source of negative pressure 14 to a boost voltage value that is higher than a nominal voltage value while the pressure level as detected by the pressure sensor 15a, 15b is above a pressure level threshold. In other words, the control circuitry 11 may be configured to not only check that the current time of day corresponds to a set period of the day before triggering the boost voltage supply, but it to also check that the pressure level is above some set pressure level threshold.

As before, the pressure level threshold may be higher than the target pressure, or analogously, the negative pressure level threshold is lower than a target negative pressure. For example, if the target pressure is -125 mmHg, the pressure level threshold may be set to -120 mmHg, -115 mmHg, or -110 mmHg. Thereby, the risk of the apparatus 10 overshooting the target pressure, and thereby risking discomfort for the patient/user, is reduced. Additionally, by utilizing a pressure level threshold as an additional condition for triggering the boost voltage, one can ensure that the boost voltage is supplied only when the apparatus 10 is in the initial depressurization state and not in the therapy regulation state.

Further, in some embodiments, and assuming that the source of negative pressure 14 is operating with the boost supply voltage, the method S300 comprises setting S303 the supply voltage to the source of negative pressure 14 to the nominal voltage value in response to the pressure level as detected by the pressure sensor 15a, 15b approaching or reaching a target pressure value. In other words, when the source of negative pressure 14 is operating at the boost voltage, the pressure level as detected by the pressure sensor 15a, 15b is monitored, and upon approaching or reaching the target pressure value, the supply voltage is reduced to the nominal voltage value. The trigger to switch the supply voltage from the boost voltage to the nominal voltage may for example be in response to the pressure level as detected by the pressure sensor 15a, 15b reaching or falling below a pressure level threshold. The pressure level threshold may accordingly be the target pressure or a pressure value above the target pressure. In reference to the latter, the pressure level threshold may be 5 mmHg above the target pressure level, 10 mmHg above the target pressure level or 15 mmHg above the target pressure level. Thereby, the source of negative pressure 14 slows down when the detected pressure level approaches the target pressure level, and the risk of overshooting the target pressure level may be reduced. Once the detected pressure level reaches the target pressure level, the method S300 may comprise deactivating S305 the source of negative pressure 14.

Moreover, in some embodiments, in the transition from the boost voltage value to the nominal voltage value, the setting S303 of the supply voltage to the nominal voltage value comprises gradually reducing S304 the supply voltage from boost voltage value to the nominal voltage value. As mentioned in the foregoing, the gradual reduction S304 of the supply voltage may be time-controlled or pressure-controlled.

Further, the method S300 may comprise one or more checks of the active time of the source of negative pressure 14, i.e., checks to see how long the source of negative pressure 14 has been on or active. The active time of the source of negative pressure may be compared to a time threshold, and in response to the active time exceeding the time threshold, the method S300 may comprise triggering S307 an alarm. For example, if the source of negative pressure 14 has been running a long time (on-time is longer than threshold), but the pressure level as detected by the pressure sensor 15a, 15b still has not reached a target pressure level, this may be indicative of a presence of a leakage condition in a single or dual lumen therapy system 1, 1' or a blockage condition in a dual lumen system 1'.

Accordingly, in some embodiments, there is provided an apparatus 10 for providing negative pressure to a wound site. The apparatus comprises a source of negative pressure 14 configured to provide negative pressure via a first fluid flow path from an inlet of the source of negative pressure to a wound site 27, and power supply circuitry 13 comprising a power source. The power supply circuitry is configured to provide a supply voltage to the source of negative pressure 14. The apparatus 10 further comprises control circuitry 11 operatively connected to the source of negative pressure 14 and the power supply circuitry 13. The control circuitry 11 is configured to, in response to detecting that a current time of day corresponds to a set period of the day, set a supply voltage level to the source of negative pressure 14 to a boost voltage value that is higher than a nominal voltage value and activate the source of negative pressure 14.

Executable instructions for performing these methods S100, S200, S300 are, optionally, included in a non-transitory computer-readable storage medium or other computer program product configured for execution by one or more processors 11 of an apparatus 10 for providing reduced pressure to a wound site 27.

The herein disclosed technology has been presented above with reference to specific embodiments. However, other embodiments than the above described are possible and within the scope of the claims. Different method steps than those described above, performing the method by hardware or software, may be provided within the scope of the claims. Thus, according to some embodiments embodiment, there is provided a non-transitory computer-readable storage medium storing one or more programs configured to be executed by one or more processors of an apparatus of a reduced-pressure wound treatment system, the one or more programs comprising instructions for performing the method according to any one of the above-discussed embodiments.

Generally speaking, a computer-accessible medium may include any tangible or non-transitory storage media or memory media such as electronic, magnetic, or optical media coupled to computer system via bus. The terms "tangible" and "non-transitory," as used herein, are intended to describe a computer-readable storage medium (or "memory") excluding propagating electromagnetic signals, but are not intended to otherwise limit the type of physical computer-readable storage device that is encompassed by the phrase computer-readable medium or memory. For instance, the terms "non-transitory computer-readable medium" or "tangible memory" are intended to encompass types of storage devices that do not necessarily store information permanently, including for example, random access memory (RAM). Program instructions and data stored on a tangible computer-accessible storage medium in non-transitory form may further be transmitted by transmission media or signals such as electrical, electromagnetic, or digital signals, which may be conveyed via a communication medium such as a network and/or a wireless link.

The processor(s) 11 (associated with the apparatus 10) may be or include any number of hardware components for conducting data or signal processing or for executing computer code stored in memory. The device 10 may accordingly have an associated memory, and the memory may be one or more devices for storing data and/or computer code for completing or facilitating the various methods described in the present description. The memory may include volatile memory or non-volatile memory. The memory may include database components, object code components, script components, or any other type of information structure for supporting the various activities of the present description. According to some embodiments, any distributed or local memory device may be utilized with the systems and methods of this description. According to some embodiments embodiment the memory is communicably connected to the processor 11 (e.g., via a circuit or any other wired, wireless, or network connection) and includes computer code for executing one or more processes described herein.

It should be noted that any reference signs do not limit the scope of the claims, that some embodiments may be at least in part implemented by means of both hardware and software, and that several "means" or "units" may be represented by the same item of hardware.

Although the figures may show a specific order of method steps, the order of the steps may differ from what is depicted. In addition, two or more steps may be performed concurrently or with partial concurrence (e.g., the setting of a specific supply voltage and the activation of the source of negative pressure). Such variation will depend on the software and hardware systems chosen and on designer choice. All such variations are within the scope of the appended claims. Likewise, software implementations could be accomplished with standard programming techniques with rule-based logic and other logic to accomplish the generating steps, activating steps, operating steps, causing steps, steps. The above mentioned and described embodiments are only given as examples and should not be limiting to the appended claims. Other solutions, uses, objectives, and functions within the scope of the below described patent claims should be apparent for the person skilled in the art.

## Claims

1. An apparatus (10) for providing negative pressure to a wound site, the apparatus comprising:
a source of negative pressure (14) configured to provide negative pressure via a first fluid flow path from an inlet of the source of negative pressure to a wound site (27);
power supply circuitry (13) comprising a power source, the power supply circuitry being configured to provide a supply voltage to the source of negative pressure (14);
control circuitry (11) operatively connected to the source of negative pressure (14) and the power supply circuitry (13);
wherein the control circuitry is configured to:
in response to receiving an indication that a noise level of ambient noise is above a noise level threshold:
set the supply voltage to the source of negative pressure (14) to a first voltage value above a nominal voltage value.

2. The apparatus (10) according to claim 1, wherein the control circuitry (11) is configured to:
in response to receiving an indication that the noise level of ambient noise is below the noise level threshold:
set the supply voltage to the source of negative pressure (14) to the nominal voltage value.

3. The apparatus (10) according to claim 1 or 2, wherein the noise level threshold is a range of noise level values, and wherein the noise level threshold for setting the supply voltage to the first voltage value is an upper value of the range of noise level values and the noise level threshold for setting the supply voltage to the nominal voltage value is a lower value of the range of noise level values.

4. The apparatus (10) according to claim any one of claims 1-3, further comprising:
a pressure sensor (15a, 15b) arranged to monitor a pressure level in a fluid flow path that is in fluid connection with the wound site;
wherein the control circuitry (11) is operatively connected to the pressure sensor and further configured to:
in response to a pressure level as detected by the pressure sensor (15a, 15b) being below a pressure level threshold and the noise level of ambient noise being above the noise level threshold:
set the supply voltage to the source of negative pressure (14) to the nominal voltage value.

5. The apparatus (10) according to claim 3 or 4, wherein the control circuitry (11) is configured to:
in transition from first voltage value to the nominal voltage value, the setting of the supply voltage to the nominal voltage value comprises gradually reducing the supply voltage from first voltage value to the nominal voltage value.

6. The apparatus (10) according to claim 5, wherein the gradual reduction of the supply voltage comprises gradually reducing the supply voltage over a set period of time.

7. The apparatus (10) according to claim 5, wherein the gradual reduction of the supply voltage comprises gradually reducing the supply voltage over a set range of pressure level values.

8. The apparatus (10) according to any one of claims 1-7, further comprising:
a microphone (70) configured to monitor the noise level of ambient noise, and wherein the control circuitry (11) is operatively connected to the microphone (70).

9. The apparatus (10) according to any one of claims 1-7, further comprising:
further comprising a communication interface (56) having at least one antenna and at least one transceiver operatively connected to the at least one antenna; and
wherein the control circuitry (11) is operatively connected to the at least one transceiver and configured to:
receive a signal comprising an indication of the noise level of ambient noise from an external handheld device (50).

10. A method (S100) for operating an apparatus (10) for providing negative pressure to a wound site, wherein the apparatus comprises a source of negative pressure (14) configured to provide negative pressure via a first fluid flow path from an inlet of the source of negative pressure to a wound site (27), and power supply circuitry (13) comprising a power source, the power supply circuitry being configured to provide a supply voltage to the source of negative pressure, wherein the method (S100) comprises:
in response to receiving an indication that a noise level of ambient noise is above a noise level threshold:
setting (S102) the supply voltage to the source of negative pressure to a first voltage value above a nominal voltage value.

11. The method (S100) according to claim 10, further comprising:
in response to receiving an indication that the noise level of ambient noise is below the noise level threshold:
setting (S103) the supply voltage to the source of negative pressure to the nominal voltage value.

12. The method (S100) according to claim 10 or 11, further comprising:
in response to a pressure level as detected by the pressure sensor being below a pressure level threshold and the noise level of ambient noise being above the noise level threshold:
setting (S102) the supply voltage to the source of negative pressure to the nominal voltage value.

13. The method (S100) according to claim 11 or 12, in transition from the first voltage value to the nominal voltage value, the setting (S103) of the supply voltage to the nominal voltage value comprises gradually reducing (S104) the supply voltage from first voltage value to the nominal voltage value.

14. A computer program product comprising instructions which, when the program is executed by a computing device of an apparatus for providing negative pressure to a wound site, causes the apparatus to carry out the method (S100) according to any one of claims 10-13.

15. A wound treatment system (1) comprising:
an apparatus (10) according to any one of claims 1-9;
a wound cover (22) for creating a sealed space defined in part by the wound site; and
a tubing assembly defining the first fluid flow path.
